# FASCICULE DE BREVET EUROPEEN

(11) **EP 0 921 126 B1**
(45) Date de publication et mention de la délivrance du brevet: **24.01.2001**
(21) Numéro de dépôt: 98402940.5
(22) Date de dépôt: 25.11.1998
(51) Int. Cl.: C07F 7/08, C07F 7/21, A61K 7/42

(54) **Dérivés siliciés de benz-x-azoles filtres, compositions cosmétiques photoprotectrices les contenant et utilisations**
Silizium enthaltende Benz-x-azolverbindungen, diese enthaltende kosmetische Sonnenschutzmittel, und Verwendungen
Silicon containing benz-x-azole compounds, cosmetic sunscreen agents containing them and their uses

(30) Priorité: 04.12.1997 FR 9715309
(43) Date de publication de la demande: 09.06.1999
(73) Titulaire: L'OREAL, 75008 Paris (FR)
(72) Inventeur: Leduc, Madeleine, 75011 Paris (FR); Richard, Hervé, 93420 Villepinte (FR); Lagrange, Alain, 77770 Coupvray (FR)
(74) Mandataire: Miszputen, Laurent

(56) Documents cités:
- EP-A- 0 712 855
- FR-A- 1 241 329

## Description

L'invention concerne de nouveaux dérivés siliciés de benz-x-azole, liposolubles, photostables et présentant un excellent pouvoir d'absorption dans le domaine des rayonnements UV. L'invention concerne également des compositions, notamment cosmétiques, contenant ces nouveaux dérivés, qui peuvent être destinées à la photoprotection de la peau et/ou des cheveux contre le rayonnement UV, en particulier le rayonnement solaire.

On sait que les radiations lumineuses de longueurs d'onde comprises entre 280 nm et 400 nm permettent le brunissement de l'épiderme humain, et que les rayons de longueurs d'onde plus particulièrement comprises entre 280 et 320 nm, connus sous la dénomination d'UV-B, provoquent des érythèmes et des brûlures cutanées qui peuvent nuire au développement du bronzage naturel. Pour ces raisons ainsi que pour des raisons esthétiques, il existe une demande croissante de moyens de contrôle de ce bronzage naturel afin de contrôler ainsi la couleur de leur peau. Il convient donc de filtrer ce rayonnement UV-B.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photo-allergiques. Ainsi, pour des raisons esthétiques et cosmétiques telles que la conservation de l'élasticité naturelle de la peau par exemple, de plus en plus de gens désirent contrôler l'effet des rayons UV-A sur leur peau. Il est donc souhaitable de filtrer aussi le rayonnement UV-A.

De nombreux composés destinés à la photoprotection (UV-A et/ou UV-B) de la peau ont été proposés à ce jour.

La plupart d'entre eux sont des composés aromatiques présentant une absorption des rayons UV dans la zone comprise entre 280 et 315 nm, ou dans la zone comprise entre 315 et 400 nm ou bien encore dans l'ensemble de ces deux zones. Ils sont le plus souvent formulés dans des compositions antisolaires qui se présentent sous la forme d'une émulsion de type huile-dans-eau (c'est à dire un support cosmétiquement acceptable constitué d'une phase continue dispersante aqueuse et d'une phase discontinue dispersée huileuse) et qui contiennent donc, à des concentrations diverses, un ou plusieurs filtres organiques classiques à fonction aromatique, lipophiles et/ou hydrophiles, capables d'absorber sélectivement les rayonnements UV nocifs, ces filtres (et leurs quantités) étant sélectionnés en fonction du facteur de protection solaire recherché (le facteur de protection solaire s'exprimant mathématiquement par le rapport du temps d'irradiation nécessaire pour atteindre le seuil érythématogène avec le filtre UV au temps nécessaire pour atteindre le seuil érythématogène sans filtre UV). Outre leur pouvoir filtrant, ces composés à activité anti-UV doivent également présenter de bonnes propriétés cosmétiques dans les compositions qui les contiennent, une bonne solubilité dans les solvants usuels et en particulier les corps gras tels que les huiles et les graisses, ainsi qu'une bonne résistance à Peau et à la transpiration (rémanence).

Parmi tous les composés aromatiques qui ont été proposés à cet effet, on peut citer les composés (hydroxy-2'-phényl)-2-benz-x-azoles de la demande de brevet CH 350 763. La solubilité de ces molécules dans différents types de formulations utilisés en matière de protection solaire reste encore insuffisante. On peut citer également les filtres UV de la demande EP-A-0712 855 qui sont des organosiloxanes ou silanes greffés par des groupes benzylidène camphre, benzimidazole ou benzotriazole ; lesdits groupes étant rélié aux atomes de silicium par un radical sulfonamidoalkylène.

La Demanderesse a découvert de manière surprenante de nouveaux dérivés siliciés de benz-x-azole présentant des propriétés améliorées, notamment au niveau de leur solubilité dans les corps gras et de leur stabilité à la lumière. Plus précisément encore, il a été trouvé, selon la présente invention, qu'en greffant sur une chaîne siliconée un ou plusieurs groupements benz-x-azole, il était possible d'aboutir à de nouveaux composés présentant, outre des propriétés filtrantes excellentes dans le domaine des rayonnements UV-A et/ou UV-B, une très bonne solubilité dans les solvants organiques usuels et notamment les corps gras tels que les huiles, ainsi que d'excellentes propriétés cosmétiques, les rendant particulièrement appropriés à une utilisation comme filtres solaires dans des, ou pour la préparation de, compositions cosmétiques destinées à la protection de la peau et/ou des cheveux contre le rayonnement ultraviolet.

La présente invention a ainsi pour premier objet de nouveaux composés qui sont caractérisés par le fait qu'ils comportent au moins une unité de formule (1) ou (2) suivante :

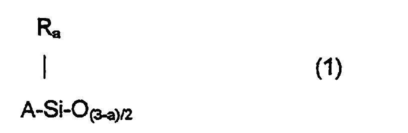

A-SiR'₁R'₂R'₃ (2)

dans lesquelles :
- R désigne un groupe hydrocarboné saturé ou insaturé en C₁-C₃₀, un groupe hydrocarboné halogéné en C₁-C₈ ou un groupe triméthylsilyloxy,
- a est égal à 1 ou 2,
- R'₁, R'₂, R'₃, identiques ou différents, sont choisis parmi les radicaux alkyles et alcényles en C₁-C₈, linéaires ou ramifiés,
   - A est un radical de formule (I) suivante : 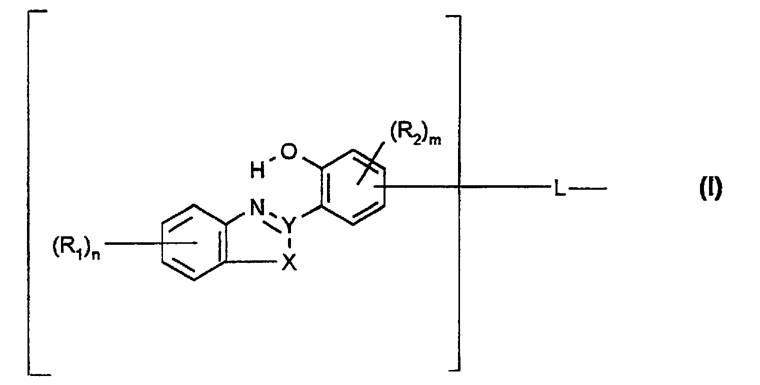
   dans laquelle :
   - L est un radical divalent permettant l'accrochage du radical A sur la chaîne siliconée;
   - les radicaux R₁ et R₂, identiques ou différents, représentent indépendamment un atome d'hydrogène, un radical alkyle en C₁-C₁₀ linéaire ou ramifié ou un radical alcènyle en C₂-C₈ linéaire ou ramifié, deux R₁ ou R₂ adjacents pouvant former ensemble un groupement alkylidène dioxy dans lequel le groupe alkylidène contient de 1 à 2 atomes de carbone,
   - Y représente C ou N ;
   - X représente O ; NR₃; S lorsque Y désigne C ou bien C lorsque Y désigne N ;
   - R₃ est un atome d'hydrogène ou un radical alkyle en C₁-C₈ ;
   - n et m sont indépendamment 1 ou 2.

De préférence, L répond à l'une des formules (a) ou (a') suivantes : 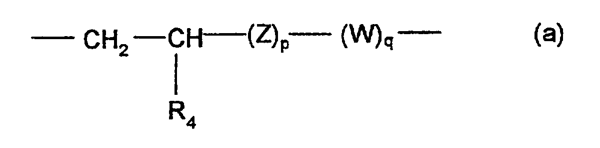 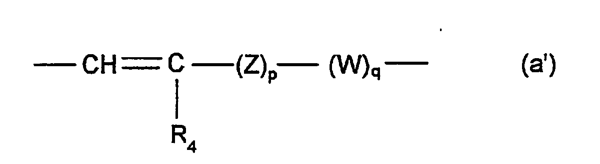 dans lesquelles :
- W représente O ou NH,
- Z est un radical alcane di-yle en C₁-C₆ linéaire ou ramifié, éventuellement substitué par un radical hydroxyle ou alcoyle en C₂-C₈, linéaire ou ramifié,
- R₄ représente un atome d'hydrogène, un radical hydroxyle ou un radical alkyle en C₁-C₈, linéaire ou ramifié,
- p et q sont 0 ou 1.

De préférence encore, les composés selon l'invention répondent à l'une des formules (3) ou (4) suivantes : 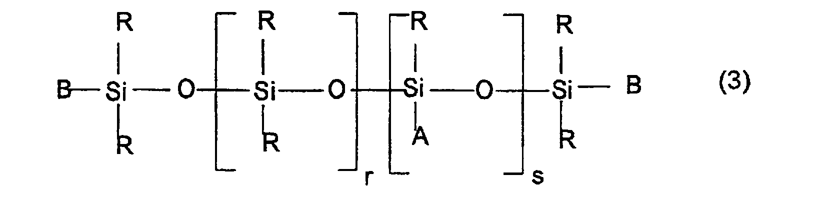 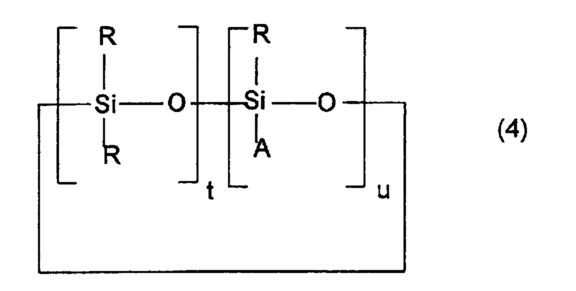 dans lesquelles :
- R désigne un groupe hydrocarboné saturé ou insaturé en C₁-C₃₀, un groupe hydrocarboné halogéné en C₁-C₈ ou un groupe triméthylsilyloxy,
- B, identiques ou différents, sont choisis parmi les radicaux R et le radical A,
- r est un nombre entier choisi entre 0 et 50 inclusivement,
- s est un nombre entier choisi entre 0 et 20 inclusivement et si s est 0, au moins un des deux symboles B est A,
- u est un nombre entier compris entre 1 et 6 indus,
- t est un nombre entier entre 0 et 10 inclus,
- t + u est égal ou supérieur à 3.

Les composés de l'invention présentent une excellente liposolubilité et peuvent ainsi être utilisés à de grandes concentrations, ce qui confère aux compositions finales des indices de protection très élevés ; par ailleurs, ils se répartissent uniformément dans les supports cosmétiques classiques contenant au moins une phase grasse ou un solvant organique cosmétiquement acceptable et peuvent être ainsi appliqués sur la peau ou les cheveux pour constituer un film protecteur efficace.

En outre, les composés de l'invention présentent un excellent pouvoir filtrant intrinsèque à l'égard des rayonnements ultraviolet UV-A et/ou UV-B.

Ces nouveaux dérivés siliciés de benz-x-azole peuvent ainsi être utilisés comme filtres solaires pour la peau humaine et les cheveux. Ils peuvent aussi être utilisés comme agents protecteurs de la lumière dans l'industrie des plastiques.

De préférence, les radicaux R, identiques ou différents sont choisis parmi les radicaux alkyles en C₁-C₁₀, linéaires ou ramifiés, le radical phényle et le radical trifluoro-3,3,3 propyle, au moins 80% en nombre des radicaux R étant le radical méthyle.

Dans les formules (1) à (4) ci-dessus, on préfère plus particulièrement les dérivés statistiques ou bien définis à blocs présentant au moins l'une des caractéristiques suivantes :
- R est méthyle,
   - B est méthyle,
   - R₁ est H,
   - R₂ est méthyle ou méthoxy,
   - p est 1,
   - q est 0 ou 1,
   - W est O
   - r est compris entre 0 et 3 inclus,
   - s est compris entre 1 et 3 inclus,
   - t + u est compris entre 3 et 5,
   - R'₁, R'₂, R'₃ sont méthyle.

Pour préparer les dérivés de formules (1) à (4). on peut procéder classiquement en mettant en oeuvre une réaction d'hydrosilylation à partir du dérivé siloxanique ou silanique correspondant dans lequel, par exemple, tous les radicaux A sont des atomes d'hydrogène. Ce dérivé est dénommé par la suite dérivé à SiH.

Les groupes SiH peuvent être présents dans la chaîne et/ou aux extrémités de la chaîne. Ces dérivés à SiH sont des produits bien connus dans l'industrie des silicones et sont généralement disponibles dans le commerce. Ils sont par exemple décrits dans les brevets américains US-A-3220972, US-A-3697473 et US-A-4340709.

Les dérivés à SiH correspondant aux composés de formules (2), (3) et (4) peuvent être donc représentés par les formules (5) à (7) suivantes :

H-SiR'₁R'₂R'₃ (5)

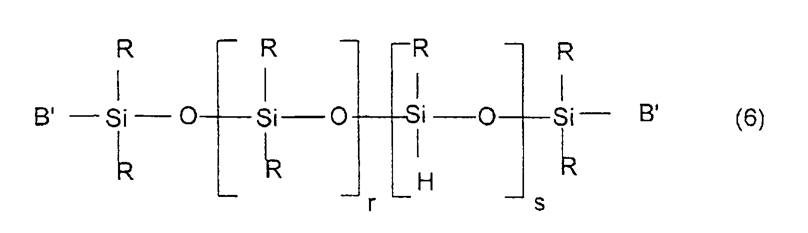

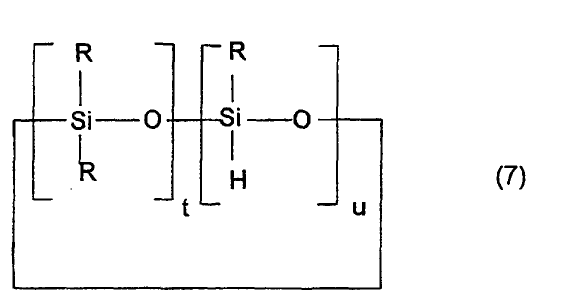

dans lesquelles :
- R'₁, R'₂ et R'₃ ont la signification donnée ci-dessus pour la formule (2),
- R, r, s, t et u ont la signification donnée ci-dessus pour les formules (3) et (4),
- B', identiques ou différents, sont choisis parmi les radicaux R et un atome d'hydrogène.

Afin de préparer les composés de l'invention de formules (2) à (4) ci-dessus, on procède de la manière suivante : sur le dérivé à SiH de formules (5), (6) ou (7), on effectue une réaction d'hydrosilylation en présence d'une quantité catalytiquement efficace d'un catalyseur au platine sur un dérivé organique de benz-x-azole choisi parmi ceux de formule (I') suivante : 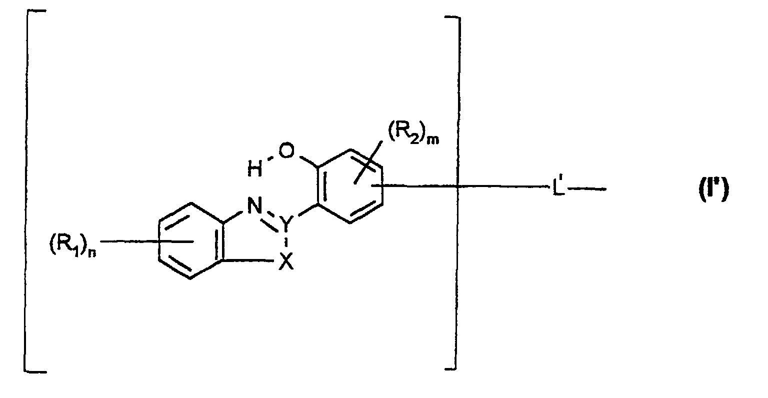 dans laquelle R₁, R₂, X, Y, n et m ont la même signification qu'à la formule (I) ci-dessus et L' répond à l'une des deux formules (b) et (b') suivantes :

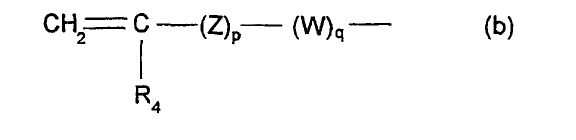

CH≡C―(Z)ₚ―(W)_{q}― (b')

dans lesquelles W, R₄, Z, p et q ont les mêmes significations qu'aux formules (a) et (a') ci-dessus.

La réaction d'hydrosilylation s'effectue donc selon l'une des deux réactions suivantes : 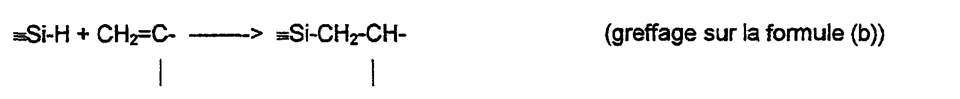 ou

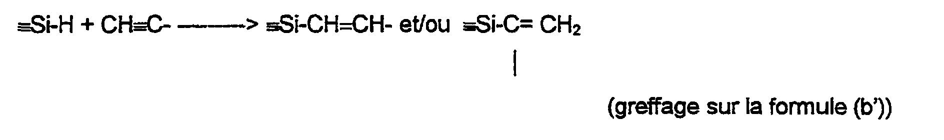

Comme dérivés de benz-x-azole utilisables pour la préparation des composés selon l'invention, on préfère tout particulièrement :
- 2-Benzoxazol-2-yl-4-methyl-6-(2-methyl-allyl)-phenol
- 2-(1H-Benzimidazol-2-yl)-4-methoxy-6-(2-methyl-allyl)-phenol

Les dérivés de formule (I') sont obtenus par condensation d'un halogénure d'alcène ou d'alcényle sur un dérivé de formule (I") 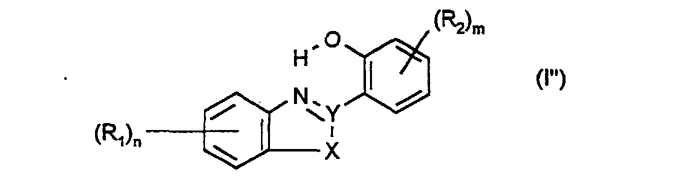 dans laquelle les radicaux R₁, R₂, X, Y, n et m ont la même signification qu'aux formules (I) et (I'), suivie par une réaction de réarrangement de Claisen.

Comme dérivés de benz-x-azole utilisables pour la préparation des composés selon l'invention de formule (I"), on préfère tout particulièrement :
- 2-Benzoxazol-2-yl-4-methyl-phenol
- 2-(1 H-Benzimidazol-2-yl)-4-methoxy-phenol

Les dérivés de formule (I") peuvent être préparés selon les modes opératoires décrits dans le brevet CH 350,763

Les dérivés silaniques de formule (2) (A-Si-R'₁R'₂R'₃) conformes à l'invention, peuvent être obtenus selon un autre procédé de synthèse qui consiste à partir du dérivé de formule (c) suivante : 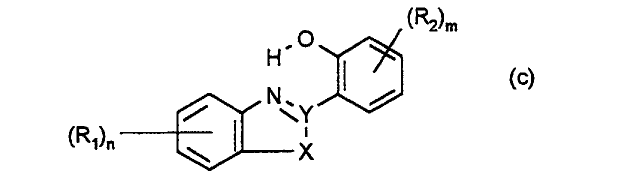 dans laquelle les radicaux R₁, R₂, X, Y, n et m ont la même signification qu'aux formules (I) et (I') ci-dessus et à lui faire réagir un dérivé silanique de formule (8) suivante :

Hal-(Z)ₚ-CHR₄-CH₂-SiR'₁R'₂R'₃ (8)

dans laquelle Hal représente un halogène et plus particulièrement le chlore et les radicaux R₄, Z, R'₁, R'₂ R'₃ et p ont les mêmes significations que ci-dessus.

Les dérivés de formule (c) peuvent être préparés selon les modes opératoires décrits dans le brevet CH 350,763

Les dérivés silaniques de formule (2) (A-Si-R'₁R'₂R'₃) conformes à l'invention, peuvent être obtenus selon un autre procédé de synthèse qui consiste à partir du dérivé de formule (9) suivante : 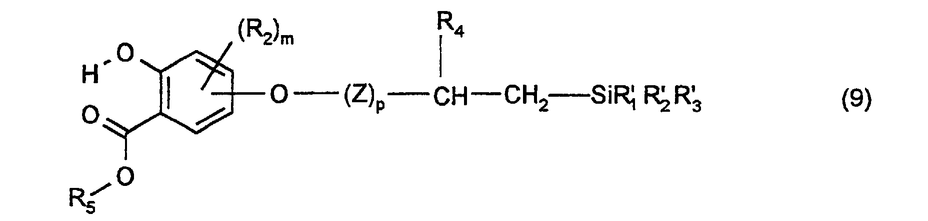 dans laquelle les radicaux R₂, R₄, Z, R'₁, R'₂ R'₃ et p ont la même signification qu'aux formules ci-dessus et R₅ est H ou méthyle, et à lui faire réagir un dérivé de formule (10) suivante: 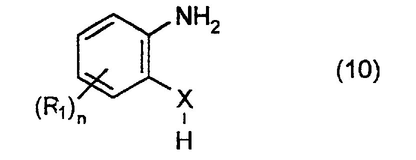 dans laquelle X a la même signification qu'aux formules ci-dessus, cette condensation cyclisation pouvant être réalisée en présence d'acide borique.

Les dérivés de formule (9) sont obtenus par condensation d'un halogénure d'alcène ou d'alcényle sur un dérivé de formule (11) suivante : 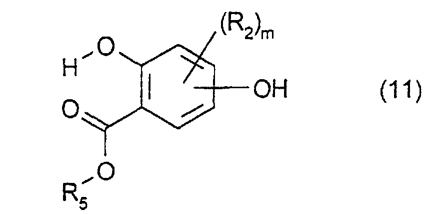 dans laquelle les radicaux R₂, R₅ et m ont la même signification qu'aux formules (I) et (9), Les produits de formules (10) et (11) sont des produits commerciaux.

La présente invention a également pour objet une composition comprenant un composé de formule (1) à (4) selon l'invention dans un support approprié. Le support peut être par exemple une composition de matière plastique. Il peut également être approprié pour une application topique. Dans ce cas, la composition selon l'invention est une composition cosmétique qui comprend un support cosmétiquement acceptable.

De préférence, la composition selon l'invention est une composition destinée à protéger une matière sensible au rayonnement ultraviolet, en particulier au rayonnement solaire, comprenant une quantité efficace d'au moins un composé conforme à l'invention. Dans une forme préférée de réalisation de l'invention, cette composition est destinée à protéger la peau et/ou les cheveux.

Les composés de formule (1), (2), (3) ou (4) sont généralement présents dans la composition de l'invention dans des proportions comprises entre 0,1 % et 20 % en poids, de préférence entre 0,5 % et 10 % en poids, par rapport au poids total de la composition.

Les compositions selon l'invention peuvent bien entendu contenir un ou plusieurs filtres solaires complémentaires actifs dans l'UVA et/ou l'UVB (absorbeurs), autres que les composés conformes à la présente invention, hydrophiles ou lipophiles. Ces filtres complémentaires peuvent être notamment choisis parmi les dérivés cinnamiques. les dérivés salicyliques, les dérivés du camphre, les dérivés de triazine, les dérivés de la benzophénone, les dérivés du dibenzoylméthane, les dérivés de β,β-diphénylacrylate, les dérivés de l'acide p-aminobenzoïque, les polymères filtres et silicones filtres décrits dans la demande WO-93/04665. D'autres exemples de filtres organiques sont donnés dans la demande de brevet EP-A-0487404.

Les compositions selon l'invention peuvent également contenir des agents de bronzage et/ou de brunissage artificiels de la peau (agents autobronzants), tels que par exemple de la dihydroxyacétone (DHA).

Les compositions selon l'invention peuvent encore contenir des pigments ou bien encore des nanopigments (taille moyenne des particules primaires : généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demande de brevets EP-A-0518772 et EP-A-0518773.

Elle peut contenir les adjuvants cosmétiques habituellement utilisés dans le domaine cosmétique, tels que des corps gras, des solvants organiques, des silicones, des épaississants, des adoucissants, des filtres solaires complémentaires, des agents anti-mousses, des agents hydratants, des parfums, des conservateurs, des tensioactifs, des charges, des séquestrants, des polymères anioniques, cationiques, non ioniques ou amphotères ou leurs mélanges, des propulseurs, des agents alcalinisants ou acidifiants, des colorants, des pigments ou nanopigments en particulier ceux destinés à assurer un effet photoprotecteur complémentaire par blocage physique du rayonnement ultraviolet, ou tout autre ingrédient habituellement utilisé en cosmétique, en particulier pour la fabrication des compositions antisolaires.

Parmi les solvants organiques, on peut citer les alcools et polyols inférieurs tels que l'éthanol, l'isopropanol, le propylèneglycol, la glycérine et le sorbitol.

Les corps gras peuvent être constitués par une huile ou par une cire ou leurs mélanges, des acides gras, des esters d'acides gras, des alcools gras, la vaseline, la paraffine, la lanoline, la lanoline hydrogénée, la lanoline acétylée. Les huiles peuvent être choisies parmi ies huiles animales, végétales, minérales ou de synthèse, et notamment l'huile de palme hydrogénée, l'huile de ricin hydrogénée, l'huile de vaseline, l'huile de paraffine. l'huile de Purcellin, les huiles de silicones, volatiles ou non, et les isoparaffines.

Bien entendu, l'homme de l'art veillera à choisir le ou les éventuels composés complémentaires cités ci-dessus et/ou leurs quantités de manière telle que les propriétés avantageuses attachées intrinsèquement au composé conforme à l'invention ne soient pas, ou substantiellement pas, altérées par la ou les adjonctions envisagées.

La composition cosmétique de l'invention peut être utilisée comme composition protectrice de l'épiderme humain ou des cheveux contre les rayons ultraviolets, comme composition antisolaire ou comme produit de maquillage.

Cette composition peut se présenter en particulier sous forme de lotion, de lotion épaissie, de gel, de crème, de lait, de poudre, de bâtonnet solide et éventuellement être conditionnée en aérosol et se présenter sous forme de mousse ou de spray.

Lorsque la composition cosmétique selon l'invention est plus particulièrement destinée à la protection de l'épiderme humain contre les rayons UV ou comme composition antisolaire, elle peut se présenter sous forme de suspension ou de dispersion dans des solvants ou des corps gras. ou encore sous forme d'émulsion (notamment de type H/E ou E/H, mais de préférence H/E) telle qu'une crème ou un lait, de dispersion vésiculaire, sous forme de pommade, de gel, de bâtonnet solide ou de mousse aérosol. Les émulsions peuvent contenir en outre des agents tensioactifs anioniques, non ioniques, cationiques ou amphotères.

Lorsque la composition cosmétique selon l'invention est utilisée pour la protection des cheveux, elle peut se présenter sous forme de shampooing, de lotion, de gel ou composition à rincer, à appliquer avant ou après shampooing, avant ou après coloration ou décoloration, avant, pendant ou après permanente ou défrisage, de lotion ou gel coiffant ou traitant, de lotion ou gel pour le brushing ou la mise en plis, de laque pour cheveux, de composition de permanente ou de défrisage, de coloration ou décoloration des cheveux.

Lorsque la composition cosmétique selon l'invention est plus particulièrement destinée à la dépigmentation de la peau, elle peut se présenter sous toutes les formes galéniques normalement utilisées pour une application topique, notamment sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide, d'une dispersion d'huile dans une phase aqueuse à l'aide de sphérules, ces sphérules pouvant être des nanoparticules polymériques telles que les nanosphères et les nanocapsules ou mieux des vésicules lipidiques de type ionique et/ou non-ionique.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau sous forme d'aérosol. Elle peut également se présenter sous forme solide, et par exemple sous forme de stick.

Lorsque la composition cosmétique selon l'invention est utilisée comme produit de maquillage des cils, des sourcils, de la peau ou des cheveux, tels que crème de traitement de l'épiderme, fond de teint, bâton de rouge à lèvres, fards à paupière, fards à joues, ligneur encore appelé "eye-liner", mascara, gel colorant, elle peut se présenter sous forme solide ou pâteuse, anhydre ou aqueuse, comme des émulsions huile-dans-eau ou eau-dans-huile, des suspensions ou encore des gels.

L'invention a encore pour objet l'utilisation d'un composé conforme à l'invention dans des, ou pour la fabrication de, compositions destinées à protéger des matières sensibles au rayonnement ultraviolet, en particulier au rayonnement solaire.

L'invention a encore pour objet l'utilisation d'un composé de formule (1), (2), (3) ou (4) conforme à l'invention pour la préparation d'un médicament destiné à prévenir les effets néfastes des rayonnements UV.

L'invention a enfin pour objet un procédé cosmétique de protection de la peau et/ou des cheveux contre le rayonnement ultraviolet, en particulier le rayonnement solaire, qui consiste à appliquer sur la peau ou les cheveux une quantité efficace de la composition cosmétique définie ci-dessus, ou d'un composé de formule (1), (2), (3) ou (4) tel que défini ci-avant.

Les exemples qui suivent illustrent l'invention sans toutefois en limiter la portée.

### EXEMPLE DE PREPARATION 1 :

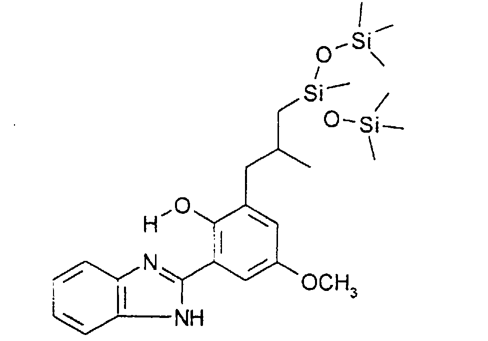

a) Première étape : préparation du 2-[5-methoxy-2-(2-méthyl-allyloxy)-phényl]-1H-benzimidazole :
Dans un mélange de 2(1H-benzimidazol-2-yl)-4-methoxy-phenol) (3.6 g, 0,015 mole) et de carbonate de potassium (2,35g, 0.017 mole) dans 20 ml de DMF porté à 85°C, on ajoute goutte à goutte en 20 minutes du chlorure de méthallyle (1,36 g, 0,015 mole). On laisse 4 heures à 85°C. On refroidit et verse le mélange réactionnel dans de l'eau glacée. Le précipité formé est filtré, lavé à l'eau et recristallisé dans le méthanol. On obtient 2,3 g (Rendement = 52 %) d'une poudre jaune pâle de 2-[5-methoxy-2-(2-méthyl-allyloxy)-phényl]-1H-benzimidazole :
- Pf 144-148 °C
b) Deuxième étape : préparation du 2-(1H-Benzimidazol-2-yl)-4-méthoxy-6-(2-méthyl-allyl)-phénol:
Le dérivé précédent (2 g) est chauffé pendant 2 heures 30 minutes à 180 °C. Après refroidissement, le mélange réactionnel est chromatographié sur silice (éluant : dichlorométhane). On obtient ainsi 2 g d'une poudre jaune pâle de 2-(1H-Benzimidazol-2-yl)-4-méthoxy-6-(2-méthyl-allyl)-phénol :
- Pf : 127-129 °C
- UV (Ethanol) λₘₐₓ = 302 nm, εₘₐₓ = 18 500
λₘₐₓ = 343 nm, εₘₐₓ = 16 200

| - Analyse élémentaire pour C₁₈ H₁₈ N₂ O₂ | | | | |
|---|---|---|---|---|
| théorie | C73.45 | H6.16 | N9.52 | O10.87 |
| trouvé | C73.34 | H6.28 | N9.52 | O11.02 |

c) Troisième étape : préparation du dérivé de l'exemple 1 :
A une solution du dérivé précédent (1,47 g, 0,005 mole) et de catalyseur (complexe à 3-3.5% poids de Pt dans du cyclovinylméthylsiloxane de Hüls Petrarch PC085 : 120 µl) dans 3 ml de toluène sec porté à 80°C, on ajoute goutte à goutte en 20 minutes 1,22 g (0,0055 mole) d'heptaméthyltrisiloxane. On laisse à cette température pendant 5 heures. On concentre le mélange réactionnel et on obtient après chromatographie sur silice (éluant : Heptane/CH₂Cl₂ 30:70) 0,5 g d'une huile incolore du dérivé de l'exemple 1 :
- UV (Ethanol) λₘₐₓ = 302 nm, εₘₐₓ = 17 780
λₘₐₓ = 343 nm, εₘₐₓ = 15 060

| - Analyse élémentaire pour C₂₅ H₄₀ N₂ O₄ Si₃ | | | | |
|---|---|---|---|---|
| théorie | C59.83 | H7.83 | N2.79 | Si16.79 |
| trouvé | C60.02 | H7.71 | N2.72 | Si16.57 |

### EXEMPLE 2 :

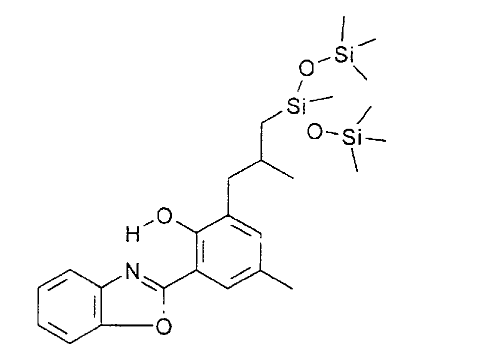

a) Première étape : préparation du 2-[5-methyl-2-(2-methyl-allyloxy)-phenyl]-benzoxazole
Dans un mélange de 2-benzoxazol-2-yl-4-methyl-phénol (5,63 g, 0,025 mole) et de carbonate de potassium (3,8 g, 0,0275 mole) dans 50 ml de DMF porté à 80°C, on ajoute goutte à goutte en 20 minutes du chlorure de méthallyle (2,49 g, 0,0275 mole). On laisse 2 heures à 80°C. On refroidit et verse le mélange réactionnel dans de l'eau glacée On extrait au dichlorométhane, sèche la phase organique et évapore le solvant. La poudre obtenue de 2-[5-methyl-2-(2-methyl-allyloxy)-phenyl]-benzoxazole (Pf : 57-58 °C) est traitée directement dans la prochaine étape.
b) Deuxième étape : préparation du 2-Benzoxazol-2-yl-4-méthyl-6-(2-methyl-allyl)-phénol
Le dérivé précédent est chauffé pendant 6 heures à 190 °C. Après refroidissement, le mélange réactionnel est chromatographié sur silice (éluant : dichlorométhane). On obtient ainsi 4,5 g d'une poudre jaune pâle de 2-Benzoxazol-2-yl-4-méthyl-6-(2-methylallyl)-phénol :
- Pf : 81-83 °C
- UV (Ethanol) λₘₐₓ = 299 nm, εₘₐₓ = 20 130
λₘₐₓ = 333 nm, εₘₐₓ = 14 180

| - Analyse élémentaire pour C₁₈ H₁₇ N O₂ | | | | |
|---|---|---|---|---|
| théorie | C77.40 | H6.13 | N5.01 | O11.46 |
| trouvé | C77.36 | H6.06 | N4.90 | O11.60 |

c) Troisième étape : préparation du dérivé de l'exemple 2
A une solution du dérivé précédent (3,8 g, 0,0136 mole) et de catalyseur (complexe à 3-3.5% poids de Pt dans du cyclovinylméthylsiloxane de Hüls Petrarch PC085 : 20 µl) dans 10 ml de toluène sec porté à 80°C, on ajoute goutte à goutte en 20 minutes 3,1 g (0,014 mole) d'heptaméthyltrisiloxane. On laisse à cette température pendant 8 heures. On concentre le mélange réactionnel et on obtient après purification par chromatographie sur colonne (éluant : Heptane/Dichlorométhane 40/60) 3.7 g (Rendement : 56%) d'une poudre blanche du dérivé de l'exemple 2 :
- Pf : 53-54 °C
- UV (Ethanol) λₘₐₓ = 287 nm, εₘₐₓ = 19 980
λₘₐₓ = 300 nm, εₘₐₓ = 24 150
λₘₐₓ = 333 nm, εₘₐₓ = 13 830

| -Analyse élémentaire pour C₂₄ H₃₇ N O₄ Si₃ | | | | |
|---|---|---|---|---|
| théorie | C59.09 | H7.65 | N2.87 | Si17.27 |
| trouvé | C58.99 | H7.85 | N2.80 | Si17.20 |

### EXEMPLE 3 :

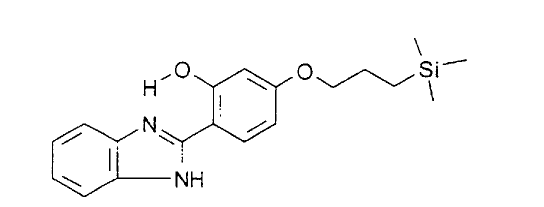

a) Première étape : préparation du 2-hydroxy-4-(3-trimethylsilanyl-propyloxy)-benzoate de méthyle
A un mélange de gentisate de méthyle (16.8 g, 0.1 mole) et de carbonate de potassium (15.2 g, 0.11 mole) dans 80 ml de DMF, sous atmosphère d'azote, à 80°C, on ajoute goutte à goutte, en 20 minutes, du chloropropyl triméthyl silane (16.6 g, 0.11 mole). On chauffe le mélange à 90°C pendant 8 heures. On refroidit et verse dans 200 ml d'eau. On extrait à l'éther diisopropylique. La phase organique est lavée à l'eau, séchée sur sulfate de sodium puis concentrée. Après chromatographie sur silice de l'huile jaune obtenue (éluant : Heptane/CH₂Cl₂ 90:10), on récupère 3.3 g d'une fraction propre de 2-hydroxy-4-(3-trimethylsilanyl-propyloxy)-benzoate de méthyle.

| - Analyse élémentaire pour C₁₄ H₂₂ O₄ Si | | | |
|---|---|---|---|
| calculé | C59.54 | H7.85 | Si9.94 |
| trouvé | C59.31 | H7.93 | Si10.20 |

b) Deuxième étape : préparation du dérivé de l'exemple 3
On porte à 170 °C sous barbotage d'azote pendant 3 heures, un mélange du dérivé précédent (2 g, 0,0071 mole), d'ortho phénylène diamine (10 g) et d'acide borique (0.05 g) dans 3 ml de N-méthyl pyrrolidone. On refroidit, verse dans l'eau le mélange réactionnel. Le précipité violet d'excès d'ortho phénylène diamine est filtré. Les jus sont concentrés et chromatographiés (éluant : dichlorométhane) pour donner le dérivé de l'exemple 3 :
- Pf : 202-208 °C
- UV (Ethanol) λₘₐₓ = 333 nm, εₘₐₓ = 22 200
λₘₐₓ = 318 nm, εₘₐₓ = 24 250

| - Analyse élémentaire pour C₁₉ H₂₄ N₂ O₂ Si : | | | | |
|---|---|---|---|---|
| calculé | C67.02 | H7.10 | N8.23 | Si8.25 |
| trouvé | C67.35 | H7.02 | N8.11 | Si8.02 |

### EXEMPLE 4 :

On donne ici un exemple concret d'une composition cosmétique conforme à l'invention, à savoir une émulsion H/E antisolaire :
- composé de l'exemple 1 4 %
- mélange d'alcool cétylstéarylique et d'alcool cétylstéarylique oyxéthyléné (33 OE) 80/20 vendu sous la dénomination commerciale « DEHSCONET 390 » par TENSIA 7 %
- mélange de mono et distéarate de glycérol vendu sous la dénomination commerciale « CERASYNTH SD » par la société ISP 2 %
- alcool cétylique 1,5 %
- polydiméthylsiloxane vendu sous la dénomination commerciale « DC200 Fluid » par la société DOW CORNING 1,5 %
- benzoate d'alcools en C₁₂-C₁₅ vendu sous la dénomination commerciale « FINSOLV TN » par la société FINETEX 16 %
- glycérine 20 %
- conservateurs qs
- eau déminéralisée qsp 100 %

Cette émulsion H/E solaire est préparée selon les techniques classiques de préparation d'émulsions en dissolvant le filtre dans la phase grasse contenant les émulsionnants, en chauffant cette phase grasse vers 70-80°C et en ajoutant, sous vive agitation, l'eau chauffée à la même température. On maintient l'agitation pendant 10 à 15 minutes, puis on laisse refroidir sous agitation modérée, et vers 40°C on ajoute enfin les conservateurs.

On obtient ainsi une crème antisolaire particulièrement efficace contre les UV B et les UV A.

## Revendications

1. Composé comportant au moins une unité de formule (1) ou (2) suivante : 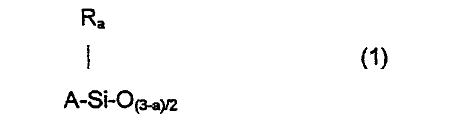
A-SiR'₁R'₂R'₃ (2)
dans lesquelles :
- R désigne un groupe hydrocarboné saturé ou insaturé en C₁-C₃₀, un groupe hydrocarboné halogéné en C₁-C₈ ou un groupe triméthylsilyloxy,
- a est égal à 1 ou 2,
- R'₁, R'₂, R'₃, identiques ou différents, sont choisis parmi les radicaux alkyles et alcényles en C₁-C₈, linéaires ou ramifiés,
- A est un radical de formule (I) suivante : 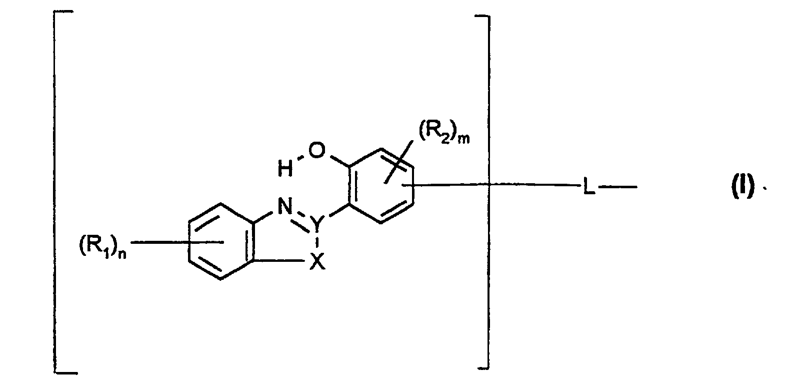
dans laquelle :
- L est un radical divalent permettant l'accrochage du radical A sur la chaîne siliconée ;
- les radicaux R₁ et R₂, identiques ou différents, représentent indépendamment un atome d'hydrogène, un radical alkyle en C₁-C₁₀ linéaire ou ramifié ou un radical alcényle en C₂-C₈ linéaire ou ramifié, deux R₁ ou R₂ adjacents pouvant former ensemble un groupement alkylidène dioxy dans lequel le groupe alkylidène contient de 1 à 2 atomes de carbone,
- Y représente C ou N ;
- X représente O ; NR₃; S lorsque Y désigne C ou bien C lorsque Y désigne N ;
- R₃ est un atome d'hydrogène ou un radical alkyle en C₁-C₈ ;
- N et M sont indépendamment 1 ou 2.

2. Composé selon la revendication 1, caractérisé par le fait que le radical L répond à l'une des formules (a) ou (a') suivantes : 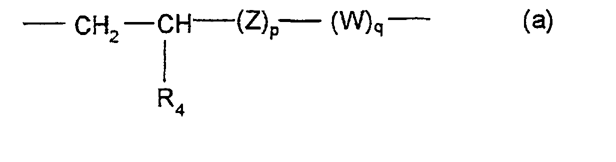 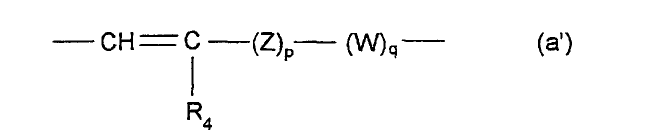 dans lesquelles :
- W représente O ou NH,
- Z est un radical alcane di-yle en C₁-C₆ linéaire ou ramifié, éventuellement substitué par un radical hydroxyle ou alcoyle en C₂-C₈, linéaire ou ramifié,
- R₄ représente un atome d'hydrogène, un radical hydroxyle ou un radical alkyle en C₁-C₈, linéaire ou ramifié,
- p et q sont 0 ou 1.

3. Composé selon l'une des revendications 1 ou 2, caractérisé par le fait qu'il répond à l'une des formules (3) ou (4) suivantes : 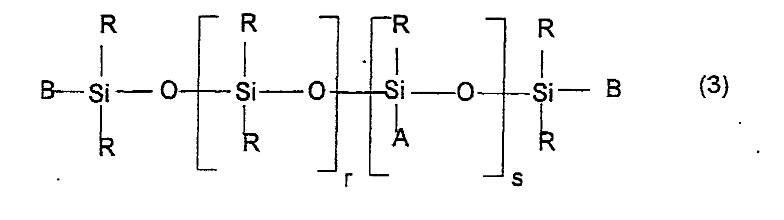 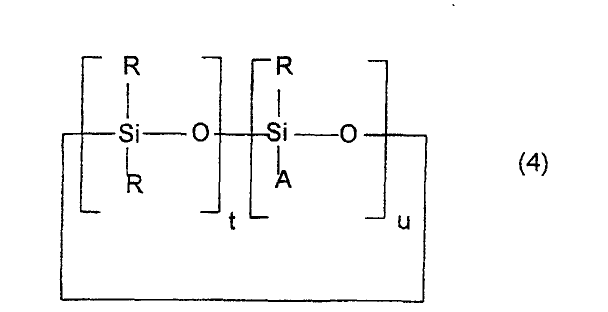 dans lesquelles :
- R désigne un groupe hydrocarboné saturé ou insaturé en C₁-C₃₀, un groupe hydrocarboné halogéné en C₁-C₈ ou un groupe triméthylsilyloxy,
- B, identiques ou différents, sont choisis parmi les radicaux R et le radical A,
- r est un nombre entier choisi entre 0 et 50 inclusivement,
- s est un nombre entier choisi entre 0 et 20 inclusivement et si s est 0, au moins un des deux symboles B est A,
- u est un nombre entier compris entre 1 et 6 inclus,
- t est un nombre entier entre 0 et 10 indus,
- t + u est égal ou supérieur à 3.

4. Composé selon la revendication 3, caractérisé par le fait qu'il répond à la formule (3) ou (4) dans laquelle les radicaux R, identiques ou différents sont choisis parmi les radicaux alkyles en C₁-C₁₀, linéaires ou ramifiés, le radical phényle et le radical trifluoro-3,3,3 propyle, et où au moins 80% en nombre des radicaux R étant le radical méthyle.

5. Composé selon la revendication 3 ou 4, caractérisé par le fait qu'il répond à l'une des formules (1) à (4) dans laquelle au moins l'une des caractéristiques suivantes est satisfaite :
- R est méthyle,
- B est méthyle,
- R₁ est H,
- R₂ est méthyle ou méthoxy,
- p est 1,
- q est 0 ou 1,
- W est O
- r est compris entre 0 et 3 inclus,
- s est compris entre 1 et 3 indus,
- t + u est compris entre 3 et 5,
- R'₁, R'₂, R'₃ sont méthyle.

6. Procédé de préparation des composés définis à l'une quelconque des revendications 1 à 5, caractérisé par le fait qu'il comprend l'étape suivante :
- sur un dérivé à SiH, on effectue une réaction d'hydrosilylation en présence d'une quantité catalytiquement efficace d'un catalyseur au platine avec un dérivé organique de benz-x-azole choisi parmi ceux de formule (I') suivante : 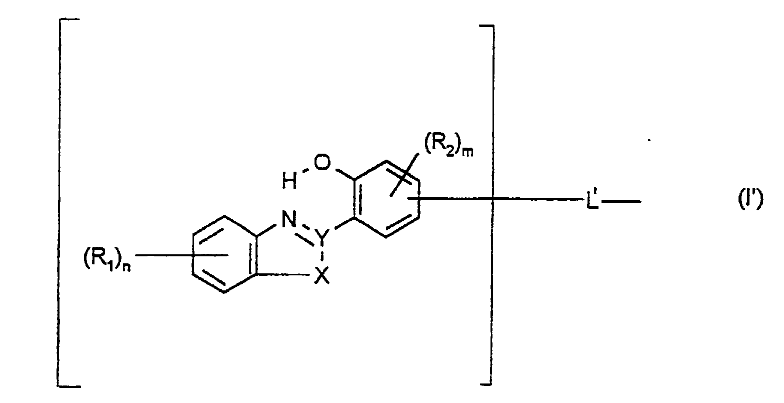 dans laquelle R₁, R₂, X, Y, n et m ont la même signification qu'à la formule (I) ci-dessus et L' répond à l'une des deux formules (b) et (b') suivantes : 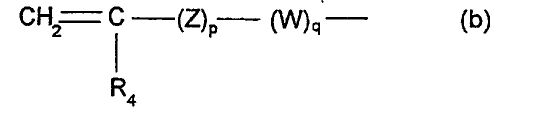
CH≡C―(Z)ₚ―(W)_{q}― (b')
dans lesquelles W, R₄, Z, p et q ont les mêmes significations dans les revendications précédentes.

7. Procédé selon la revendication 6, caractérisé par le fait que le dérivé SiH est un composé répondant à l'une des formules (5) à (7) suivantes :
H-SiR'₁R'₂R'₃ (5) 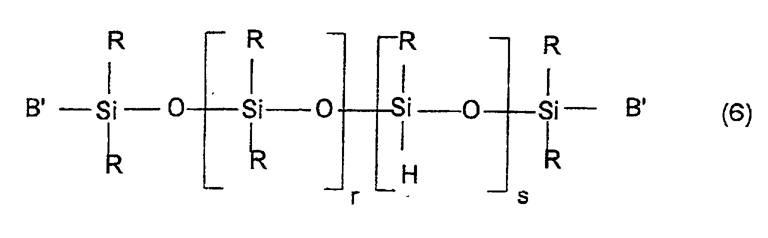 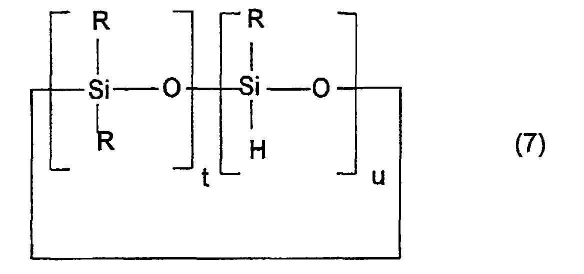
dans lesquelles :
- R'₁, R'₂ et R'₃ ont la signification donnée à la revendication 1 pour la formule (2),
- R, r, s, t et u ont la signification donnée à la revendication 3 pour les formules (3) et (4),
- B', identiques ou différents, sont choisis parmi les radicaux R et un atome d'hydrogène.

8. Procédé de préparation des composés définis par la formule (2) à la revendication 1, caractérisé par le fait qu'il consiste à faire réagir un dérivé de formule (c) suivante : 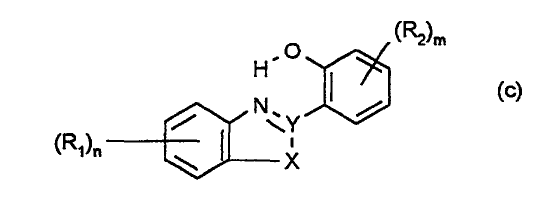 dans laquelle les radicaux R₁, R₂, X, Y, n et m ont la même signification qu'aux formules (I) et (I') ci-dessus avec un dérivé silanique de formule (8) suivante :
Hal-(Z)ₚ-CHR₄-CH₂-SiR'₁R'₂R'₃ (8)
dans laquelle Hal représente un halogène et plus particulièrement le chlore et les radicaux R₄, Z, R'₁, R'₂ R'₃ et p ont les mêmes significations que ci-dessus.

9. Procédé de préparation des composés définis par la formule (2) à la revendication 1, caractérisé par le fait qu'il comprend l'étape suivante :
sur un dérivé de formule (9) suivante : 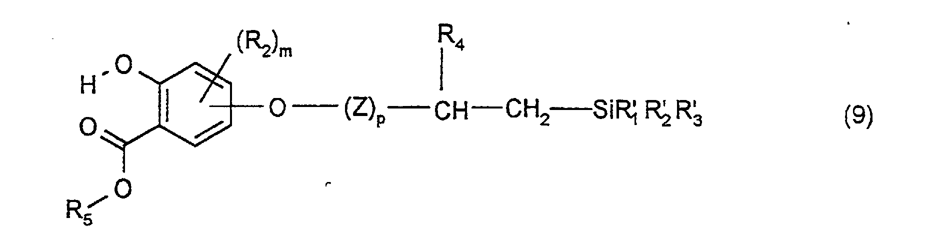 dans laquelle les radicaux R₂, R₄, Z, R'₁, R'₂ R'₃ et p ont les mêmes significations indiquées dans la revendication 1 et R₅ est H ou méthyle, on fait réagir un dérivé de formule (10) suivante : 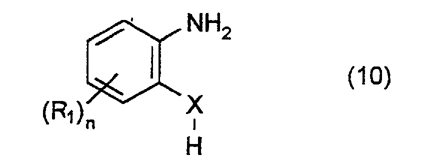 dans laquelle X a la même signification indiquée dans la revendication 1, cette condensation cyclisation pouvant être réalisée en présence d'acide borique.

10. Composition cosmétique comprenant dans un support cosmétiquement acceptable au moins un composé tel que défini à l'une quelconque des revendications 1 à 5.

11. Composition selon la revendication 10, caractérisée par le fait que le composé tel que défini à l'une quelconque des revendications 1 à 5 est présent dans la composition à une teneur allant de 0,1 à 20 % en poids, par rapport au poids total de la composition, de préférence allant de 0,5 % à 10 % en poids, par rapport au poids total de la composition.

12. Composition de matière plastique comprenant au moins un composé tel que défini à l'une quelconque des revendications 1 à 5.

13. Utilisation d'au moins un composé tel que défini à l'une quelconque des revendications 1 à 5 dans une, ou pour la fabrication d'une, composition destinée à protéger des matières sensibles au rayonnement ultraviolet, en particulier au rayonnement solaire.

14. Utilisation selon la revendication 13, où la composition est une formulation cosmétique destinée à protéger la peau ou les cheveux du rayonnement ultraviolet, en particulier du rayonnement solaire.

15. Utilisation selon la revendication 14, où la composition est une composition de matière plastique.

16. Utilisation d'au moins un composé tel que défini à l'une quelconque des revendications 1 à 5 pour la préparation d'un médicament destiné à prévenir les effets néfastes des rayonnements UV.

## Patentansprüche

1. Verbindung, die mindestens eine Einheit der folgenden Formeln (1) oder (2) aufweist: 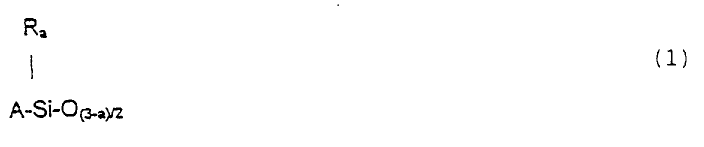
A-SiR'₁R'₂R'₃ (2),
worin:
- R eine gesättigte oder ungesättigte C₁₋₃₀-Kohlenwasserstoffgruppe, eine halogenierte C₁₋₈-Kohlenwasserstoffgruppe oder eine Trimethylsilyloxygruppe bedeutet,
- a 1 oder 2 ist,
- die Gruppen R'₁, R'₂ und R'₃, die identisch oder voneinander verschieden sind, unter den geradkettigen oder verzweigten Alkyl- und Alkenylgruppen mit 1 bis 8 Kohlenstoffatomen ausgewählt sind, und
- A eine Gruppe der folgenden Formel (I) ist: 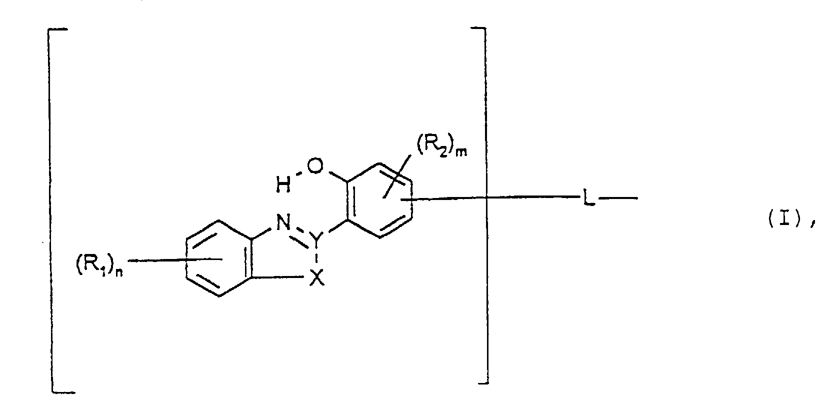
worin bedeuten:
- L eine zweiwertige Gruppe, über die die Gruppe A an die Siliconkette gebunden werden kann,
- die Gruppen R₁ und R₂, die identisch oder voneinander verschieden sind, unabhängig voneinander Wasserstoff, eine geradkettige oder verzweigte C₁₋₁₀-Alkylgruppe oder eine geradkettige oder verzweigte C₂₋₈-Alkenylgruppe, wobei zwei angrenzende Gruppen R₁ oder R₂ gemeinsam eine Alkylidendioxygruppe bilden können, wobei die Alkylidengruppe 1 bis 2 Kohlenstoffatome aufweist,
- Y C oder N,
- X O; NR₃; S, wenn Y C bedeutet, oder C, wenn Y N bedeutet,
- R₃ Wasserstoff oder eine C₁₋₈-Alkylgruppe,
- n und m unabhängig voneinander 1 oder 2.

2. Verbindung nach Anspruch 1, dadurch gekennzeichnet, daß die Gruppe L vorzugsweise einer der folgenden Formeln (a) oder (a') entspricht: 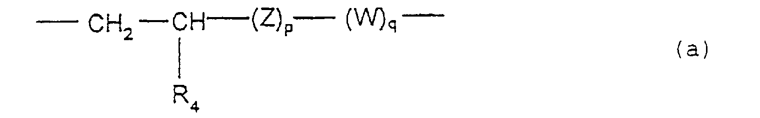 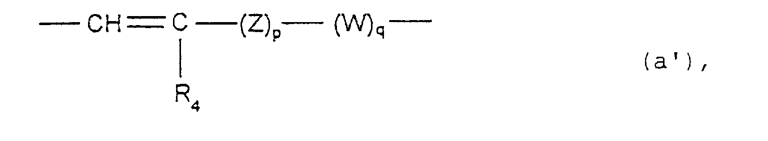 worin bedeuten:
- W O oder NH,
- Z eine geradkettige oder verzweigte C₁₋₆-Alkandiylgruppe, die gegebenenfalls mit einer Hydroxygruppe oder einer geradkettigen oder verzweigten C₂₋₈-Alkylgruppe substituiert ist,
- R₄ Wasserstoff, Hydroxy oder eine geradkettige oder verzweigte C₁₋₈-Alkylgruppe, und
- p und q 0 oder 1.

3. Verbindung nach einem der Ansprüche 1 oder 2, dadurch gekennzeichnet, daß sie einer der folgenden Formeln (3) oder (4) entspricht: 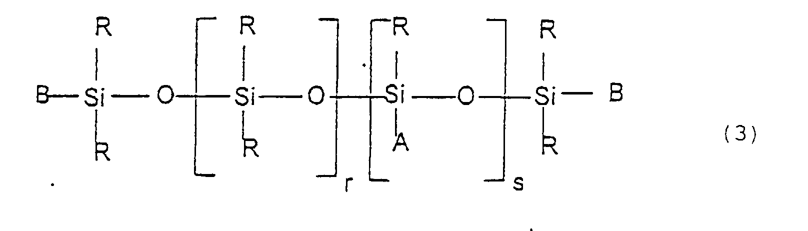 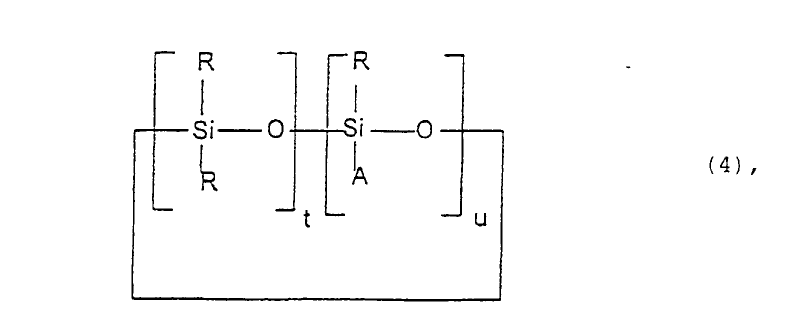 worin:
- die Gruppen R eine gesättigte oder ungesättigte C₁₋₃₀-Kohlenwasserstoffgruppe, eine halogenierte C₁₋₈-Kohlenwasserstoffgruppe oder eine Trimethylsilyloxygruppe bedeuten,
- die Gruppen B, die identisch oder voneinander verschieden sind, unter den Gruppen R und der Gruppe A ausgewählt sind,
- r Null oder eine ganze Zahl im Bereich von 1 bis 50 bedeutet,
- s Null oder eine ganze Zahl im Bereich 1 bis 20 bedeutet, wobei mindestens eine der beiden Gruppen B A bedeutet, wenn s Null ist,
- u eine ganze Zahl im Bereich von 1 bis 6 bedeutet,
- t Null oder eine ganze Zahl im Bereich von 1 bis 10 bedeutet, und
- t + u mindestens 3 ist.

4. Verbindung nach Anspruch 3, dadurch gekennzeichnet, daß sie der Formel (3) oder (4) entspricht, worin die Gruppen R, die identisch oder voneinander verschieden sind, unter den geradkettigen oder verzweigten C₁₋₁₀-Alkylgruppen, Phenyl und 3,3,3-Trifluorpropyl ausgewählt sind, wobei mindestens 80 % der Gruppen R Methyl bedeuten.

5. Verbindung nach Anspruch 3 oder 4, dadurch gekennzeichnet, daß sie einer der Formeln (1) bis (4) entspricht, für die mindestens eine der folgenden Bedingungen erfüllt ist:
- R ist Methyl;
- B ist Methyl;
- R₁ ist H;
- R₂ ist Methyl oder Methoxy;
- p ist 1;
- q ist 0 oder 1;
- W ist O;
- r liegt im Bereich von 0 bis 3;
- s liegt im Bereich von 1 bis 3;
- t + u liegt im Bereich von 3 bis 5; und
- R'₁, R'₂ und R'₃ bedeuten Methyl.

6. Verfahren zur Herstellung der Verbindungen nach einem der Ansprüche 1 bis 5, gekennzeichnet durch den folgenden Schritt:
- in Gegenwart einer katalytisch wirksamen Menge eines Platinkatalysators wird eine Hydrosilylierungsreaktion mit einem SiH-Derivat und einem organischen Benz-x-azolderivat durchgeführt, das unter den Derivaten der folgenden Formel (I') ausgewählt ist: 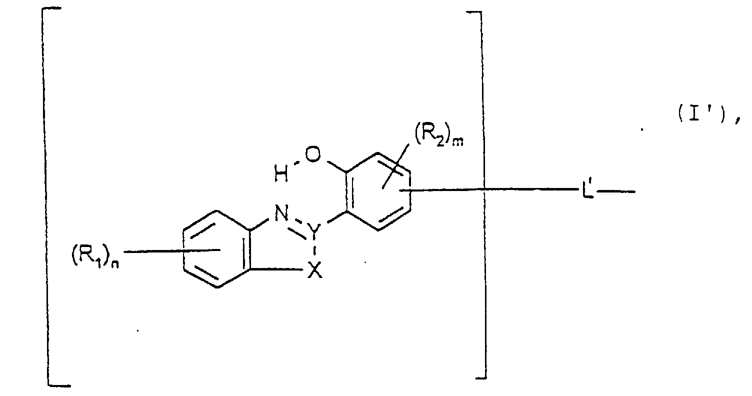 worin R₁, R₂, X, Y, n und m die für die Formel (I) angegebenen Bedeutungen aufweisen und worin L' einer der beiden folgenden Formeln (b) und (b') entspricht: 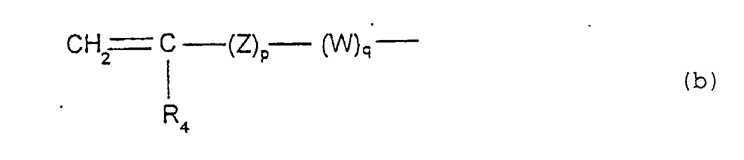
CH≡C―(Z)ₚ―(W)_{q}― (b'),
worin die Gruppen W, R₄, Z, p und q die oben in den vorhergehenden Ansprüchen angegebenen Bedeutungen aufweisen.

7. Verfahren nach Anspruch 6, dadurch gekennzeichnet-, daß das SiH-Derivat eine Verbindung ist, die einer der folgenden Formeln (5) bis (7) entspricht:
H-SiR'₁R'₂R'₃ (5) 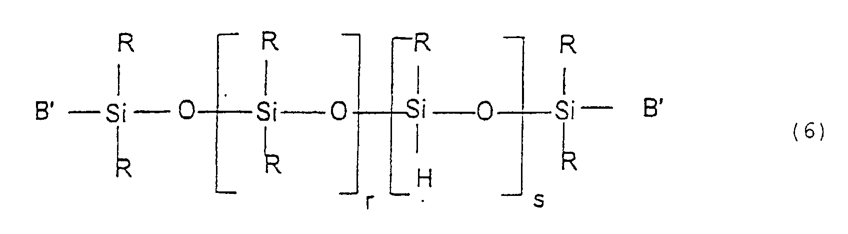 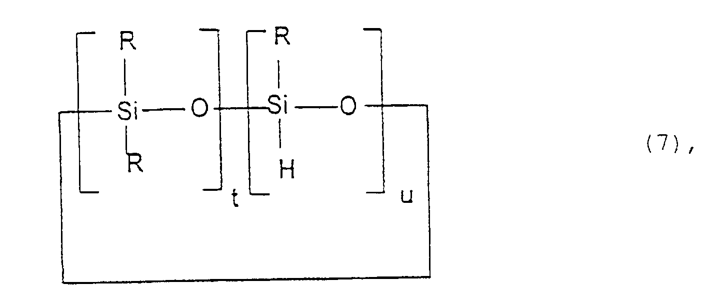
worin:
- R'₁, R'₂ und R'₃ die in Anspruch 1 für die Formel (2) angegebenen Bedeutungen aufweisen,
- R, r, s, t und u die in Anspruch 3 für die Formeln (3) und (4) angegebenen Bedeutungen aufweisen, und
- die Gruppen B', die identisch oder voneinander verschieden sind, unter den Gruppen R und Wasserstoff ausgewählt sind.

8. Verfahren zur Herstellung von Verbindungen der Formel (2) nach Anspruch 1, dadurch gekennzeichnet, daß es darin besteht, ein Derivat der folgenden Formel (c): 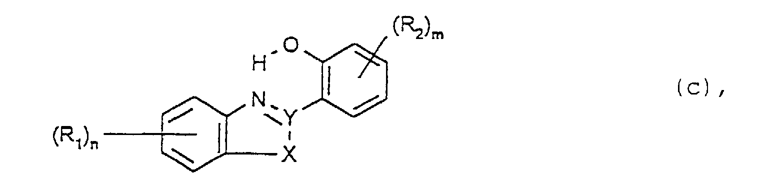 worin die Gruppen R₁, R₂, X, Y, n und m die für die Formeln (I) und (I') angegebenen Bedeutungen aufweisen, mit einem Silanderivat der folgenden Formel (8) umzusetzen:
Hal-(Z)ₚ-CHR₄-CH₂-Si-R'₁R'₂R'₃ (8),
worin Hal Halogen und insbesondere Chlor bedeutet und die Gruppen R₄, Z, R'₁, R'₂, R'₃ und p die oben genannten Bedeutungen aufweisen.

9. Verfahren zur Herstellung von Verbindungen der Formel (2) nach Anspruch 1, dadurch gekennzeichnet, das darin besteht, ein Derivat der folgenden Formel (9): 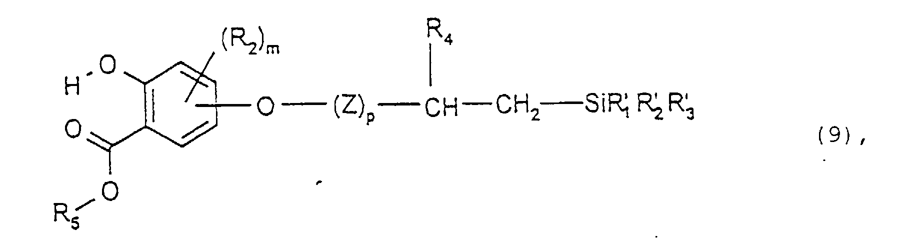 worin die Gruppen R₂, R₄, Z, R'₁, R'₂, R'₃ und p die oben in Anspruch 1 angegebenen Bedeutungen aufweisen und R₅ H oder Methyl bedeutet, mit einem Derivat der folgenden Formel (10) umzusetzen: 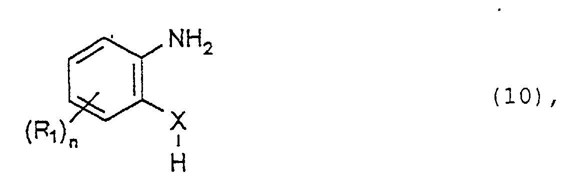 worin X die in Anspruch 1 angegebenen Bedeutungen aufweist, wobei die Kondensation/Cyclisierung in Gegenwart von Borsäure durchgeführt werden kann.

10. Kosmetische Zusammensetzung, die in einem kosmetisch akzeptablen Träger mindestens eine Verbindung nach einem der Ansprüche 1 bis 5 enthält.

11. Zusammensetzung nach Anspruch 10, dadurch gekennzeichnet, daß die Verbindung nach einem der Ansprüche 1 bis 5 in der Zusammensetzung in einem Mengenanteil von 0,1 bis 20 Gew.-%, bezogen auf das Gesamtgewicht der Zusammensetzung, und vorzugsweise im Bereich von 0,5 bis 10 Gew.-%, bezogen auf das Gesamtgewicht der Zusammensetzung, vorliegt.

12. Zusammensetzung aus Kunststoff, die mindestens eine Verbindung nach einem der Ansprüche 1 bis 5 enthält.

13. Verwendung mindestens einer Verbindung nach einem der Ansprüche 1 bis 5 in einer Zusammensetzung, die dazu dienen soll, gegenüber UV-Strahlung und insbesondere gegenüber Sonnenlicht empfindliche Materialien zu schützen, oder zu deren Herstellung.

14. Verwendung nach Anspruch 13, wobei die Zusammensetzung eine kosmetische Formulierung ist, die zum Schutz der Haut und/oder der Haare gegen UV-Strahlung und insbesondere gegenüber Sonnenlicht dienen soll.

15. Verwendung nach Anspruch 14, wobei die Zusammensetzung eine Zusammensetzung aus Kunststoff ist.

16. Verwendung mindestens einer Verbindung nach einem der Ansprüche 1 bis 5 zur Herstellung eines Arzneimittels, das den schädlichen Wirkungen der UV-Strahlung vorbeugen soll.

## Claims

1. Compound comprising at least one unit of formula (1) or (2) below: 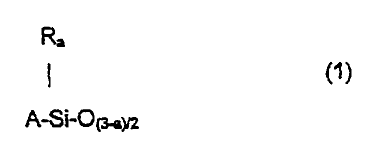
A-SiR'₁R'₂R'₃ (2)
in which:
- R denotes a saturated or unsaturated C₁-C₃₀ hydrocarbon-based group, a halogenated C₁-C₈ hydrocarbon-based group or a trimethylsilyloxy group,
- a is equal to 1 or 2,
- R'₁, R'₂ and R'₃, which may be identical or different, are chosen from linear or branched C₁-C₈ alkyl and alkenyl radicals,
- A is a radical of formula (I) below: 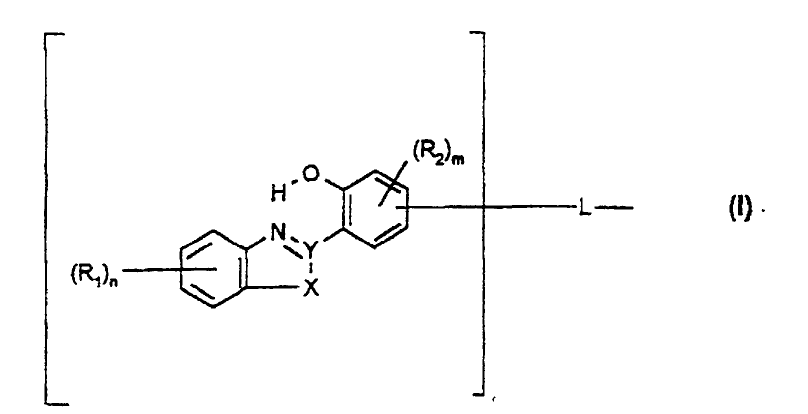
in which:
- L is a divalent radical for attaching the radical A to the silicone chain;
- the radicals R₁ and R₂, which may be identical or different, independently represent a hydrogen atom, a linear or branched C₁-C₁₀ alkyl radical or a linear or branched C₂-C₈ alkenyl radical, it being possible for two adjacent radicals R₁ or R₂ together to form an alkylidenedioxy group in which the alkylidene group contains 1 or 2 carbon atoms,
- Y represents C or N;
- X represents O; NR₃; S when Y denotes C or else C when Y denotes N;
- R₃ is a hydrogen atom or a C₁-C₈ alkyl radical;
- n and m are, independently, 1 or 2.

2. Compound according to Claim 1, characterized in that the radical L corresponds to either of the formulae (a) and (a') below: 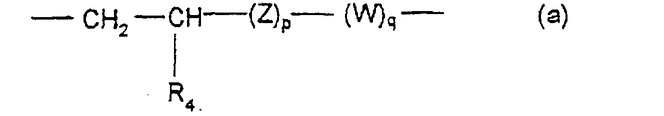 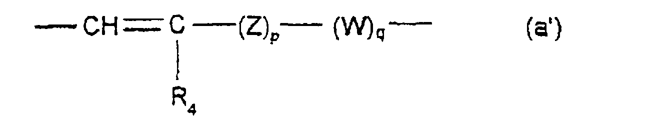 in which:
- W represents O or NH,
- Z is a linear or branched C₁-C₆ alkanediyl radical optionally substituted with a hydroxyl or linear or branched C₂-C₈ alkyl radical,
- R₄ represents a hydrogen atom, a hydroxyl radical or a linear or branched C₁-C₈ alkyl radical,
- p and q are 0 or 1.

3. Compound according to either of Claims 1 and 2, characterized in that it corresponds to either of the formulae (3) and (4) below: 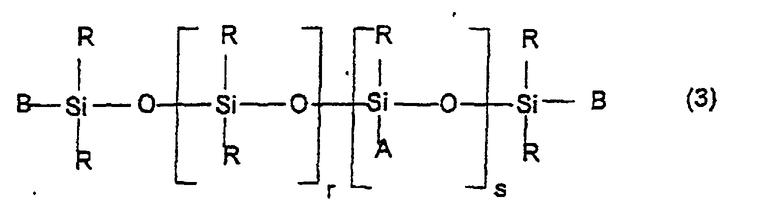 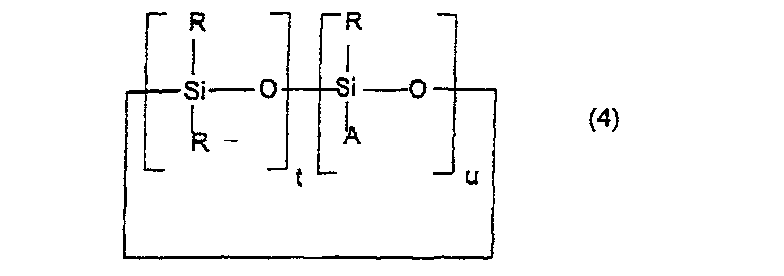 in which:
- R denotes a saturated or unsaturated C₁-C₃₀ hydrocarbon-based group, a halogenated C₁-C₈ hydrocarbon-based group or a trimethylsilyloxy group,
- B, which may be identical or different, are chosen from the radicals R and the radical A,
- r is an integer chosen between 0 and 50 inclusive,
- s is an integer chosen between 0 and 20 inclusive and if s is 0, at least one of the two symbols B is A,
- u is an integer between 1 and 6 inclusive,
- t is an integer between 0 and 10 inclusive,
- t + u is greater than or equal to 3.

4. Compound according to Claim 3, characterized in that it corresponds to formula (3) or (4) in which the radicals R, which may be identical or different, are chosen from linear or branched C₁-C₁₀ alkyl radicals, a phenyl radical and a 3,3,3-trifluoropropyl radical, and in which at least 80%, in numerical terms, of the radicals R are methyl radicals.

5. Compound according to Claim 3 or 4, characterized in that it corresponds to one of the formulae (1) to (4) in which at least one of the following characteristics is satisfied:
- R is methyl,
- B is methyl,
- R₁ is H,
- R₂ is methyl or methoxy,
- p is 1,
- q is 0 or 1,
- W is O,
- r is between 0 and 3 inclusive,
- s is between 1 and 3 inclusive,
- t + u is between 3 and 5,
- R'₁, R'₂ and R'₃ are methyl.

6. Process for preparing the compounds defined in any one of Claims 1 to 5, characterized in that it comprises the following step: - a hydrosilylation reaction is carried out on a derivative containing SiH, in the presence of a catalytically effective amount of a platinum catalyst with an organic benz-x-azole derivative chosen from those of formula (I') below: 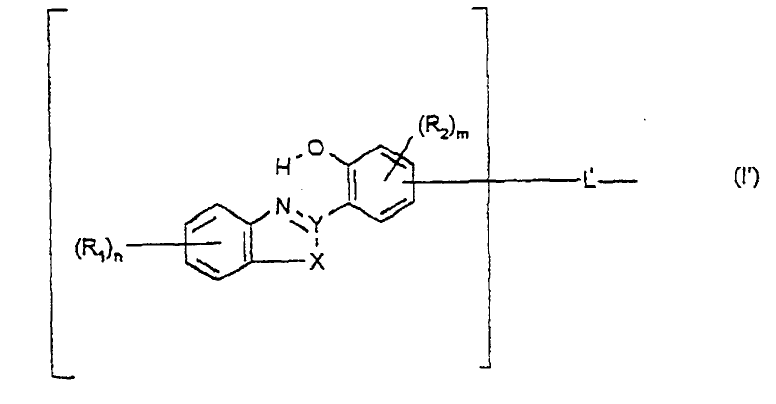 in which R₁, R₂, X, Y, n and m have the same meaning as in formula (I) above and L' corresponds to one of the two formulae (b) and (b') below: 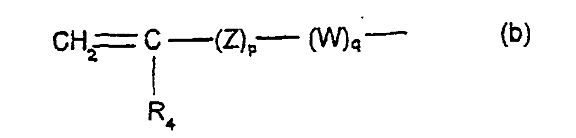
CH≡C―(Z)ₚ―(W)_{q}― (b')
in which W, R₄, Z, p and q have the same meanings as in the preceding claims.

7. Process according to Claim 6, characterized in that the SiH derivative is a compound corresponding to one of the formulae (5) to (7) below:
H-SiR'₁R'₂R'₃ (5) 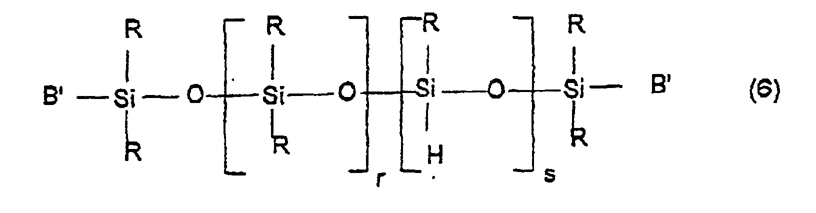 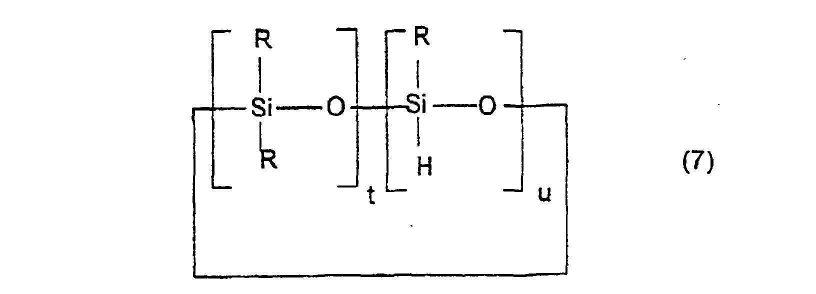
in which:
- R'₁, R'₂ and R'₃ have the meaning given in Claim 1 for formula (2),
- R, r, s, t and u have the meaning given in Claim 3 for formulae (3) and (4),
- B', which may be identical or different, are chosen from the radicals R and a hydrogen atom.

8. Process for preparing the compounds defined by formula (2) in Claim 1, characterized in that it consists in reacting a derivative of formula (c) below: 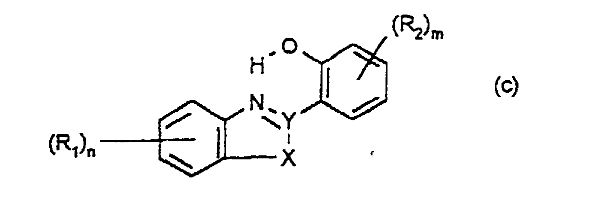 in which the radicals R₁, R₂, X, Y, n and m have the same meaning as in formulae (I) and (I') above, with a silane derivative of formula (8) below:
Hal-(Z)ₚ-CHR₄-CH₂-SiR'₁R'₂R'₃ (8)
in which Hal represents a halogen and more particularly chlorine and the radicals R₄, Z, R'₁, R'₂, R'₃ and p have the same meanings as above.

9. Process for preparing the compounds defined by formula (2) in Claim 1, characterized in that it comprises the following step:
a derivative of formula (9) below: 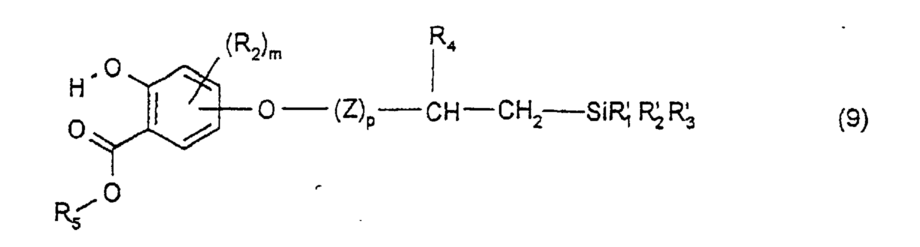 in which the radicals R₂, R₄, Z, R'₁, R'₂, R'₃ and p have the same meanings indicated in Claim 1 and R₅ is H or methyl, is reacted with a derivative of formula (10) below: 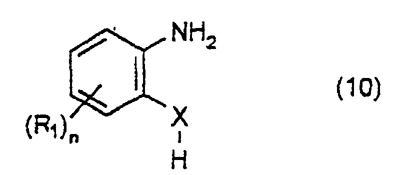 in which X has the same meaning indicated in Claim 1, it being possible for this cyclizing condensation to be carried out in the presence of boric acid.

10. Cosmetic composition comprising, in a cosmetically acceptable support, at least one compound as defined in any one of Claims 1 to 5.

11. Composition according to Claim 10, characterized in that the compound as defined in any one of Claims 1 to 5 is present in the composition in a content ranging from 0.1 to 20% by weight relative to the total weight of the composition, preferably ranging from 0.5% to 10% by weight relative to the total weight of the composition.

12. Plastics composition comprising at least one compound as defined in any one of Claims 1 to 5.

13. Use of at least one compound as defined in any one of Claims 1 to 5, in, or for the manufacture of, a composition intended for protecting materials that are sensitive to ultraviolet light, in particular to solar radiation.

14. Use according to Claim 13, in which the composition is a cosmetic formulation intended to protect the skin or the hair against ultraviolet radiation, in particular solar radiation.

15. Use according to Claim 14, in which the composition is a plastics composition.

16. Use of at least one compound as defined in any one of Claims 1 to 5, for the preparation of a medicinal product intended for preventing the harmful effects of UV radiation.
